Europäisches Patentamt

European Patent Office

Office européen des brevets

(11) Publication number: **0 211 639 B1**

## EUROPEAN PATENT SPECIFICATION

(45) Date of publication of patent specification: **29.12.93**

(51) Int. Cl.⁵: **C12N 15/00**, C12N 9/82, C12N 1/20, //(C12N1/20, C12R1:18,1:19,1:40)

(21) Application number: **86305984.6**

(22) Date of filing: **04.08.86**

(54) Production of L-asparaginase.

(30) Priority: **06.08.85 GB 8519753**

(43) Date of publication of application:
**25.02.87 Bulletin 87/09**

(45) Publication of the grant of the patent:
**29.12.93 Bulletin 93/52**

(84) Designated Contracting States:
**AT BE CH DE FR GB IT LI LU NL SE**

(56) References cited:
**DE-A- 1 942 833**

**CHEMICAL ABSTRACTS, vol. 76, no. 5, 10th April 1972, page 180, abstract no.82845k, Columbus, Ohio, US; K.A. CAMMACK et al.: "Physical properties andsubunit structure of L-asparaginase isolated from Erwinia carotovora",& BIOCHEM. J. 1972, 126(2), 361-79**

**IDEM**

(73) Proprietor: **The Public Health Laboratory Service Board**
**61 Colindale Avenue**
**London NW9 5EO(GB)**

(72) Inventor: **Atkinson, Anthony**
**"Twingley"**
**Mill Corner**
**Winterbourne**
**Gunner Salisbury Wiltshire(GB)**
Inventor: **Minton, Nigel Peter**
**Thatch End**
**16 Newton Tony**
**Salisbury Wiltshire(GB)**
Inventor: **Gilbert, Harold John**
**16 Kells Gardens**
**Low Fell**
**Stateshead Tyne and Wear(GB)**

(74) Representative: **Ritter, Stephen David et al**
**Mathys & Squire**
**10 Fleet Street**
**London EC4Y 1AY (GB)**

CHEMICAL ABSTRACTS, vol. 92, no. 12, 9th June 1980, page 262, abstract no.193476r, Columbus, Ohio, Us; T. MAITA et al.: "The primary structure of L-asparaginase from Escherichia coli", & HOPPE-SEYLER'S Z. PHYSIOL.CHEM. 1980, 361(2), 105-17

IDEM

CHEMICAL ABSTRACTS, vol. 89, no. 5, 25th September 1978, page 377, abstract no.103154v, Columbus, Ohio, US; S. BROOME et al.: "Immunological screening methodto detect specific translation products",& PROC. NATL. ACAD. SCI. U.S.A. 1978, 75(6), 2746-9

P.H. POUWELS et al.: "Cloning vectors", 1985, pages I-A-iv-20,II-A-a-i-5,Elsevier Science Publishers B.V., Amsterdam, NL

**Description**

This invention relates to L-asparaginase and to a process for producing it by culturing micro-organisms containing genetic material produced by recombinant DNA techniques.

L-Asparaginase (EC 3.5.1.1.; L-asparagine amidohydrolase) catalyses the deamination of L-asparagine to L-aspartate and ammonia. The enzyme has been isolated from a variety of sources, occurring in animal (Broome, 1961) and plant cells (Sokek et al., 1980), yeasts (Jones, 1977), fungi (Arst and Bailey, 1980) and bacteria (Wade et al., 1980).

The enzyme is produced commercially as a therapeutic product for treating malignant conditions, including certain types of leukaemia and disseminated cancer.

Extensively used in the treatment of accute lymphatic leukaemia (Broome, 1961), the enzyme causes a plasma depletion of circulatory L-asparagine to which lymphatic carcinomas are particularly sensitive.

The enzyme is typically produced by procedures such as those described in UK Patent Specification Nos. 1258062, 1258063, 1314530 and 1379728 by culturing micro-organisms of the genus Erwinia in defined culture media. In the following description the abbreviation 'EA' designates L-asparaginase.

To date the principal source of asparaginase has been the bacterium Erwinia chrysanthemi NCPPB 1066. This particular enzyme is tetrameric with a subunit molecular weight of $32 \times 10^3$. Studies on the regulation of asparaginase production in E. chrysanthemi indicate that synthesis of the protein is repressed by both glucose and glycerol, but is unaffected by both nitrogen and aspartate (Callow et al., 1971). Asparagine does not act as an inducer of asparaginase synthesis.

Considerable effort has been devoted to the selection of strains of micro-organism which are capable of producing L-asparaginase in high yields. However, even the best strains currently available, namely Erwinia carotovora SCI 193 and Erwinia chrysanthemi NCPB 1066 yield quantities of L-asparaginase which are lower than would be desirable.

We have now surprisingly discovered that by producing recombinant plasmids containing genetic material coding for L-asparaginase and using the obtained plasmids to transform host micro-organisms, transformed micro-organisms can be obtained which are capable of producing in high yield enzymes having L-asparaginase activity. Furthermore, the degree of variation of yield has been found to be less than in currently used production stains.

Although the reason for the superior performance of the strains produced in accordance with the invention has not been established with certainty, it is believed to be at least in part due to the absence in the transformed strains of high levels of proteases which are present in the production strains. A further factor which possibly contributes to the superiority of transformed strains according to the invention is that recombinant plasmids containing genetic material coding for L-asparaginase may be present in the transformed micro-organisms in plural copies.

Thus according to one aspect of the present invention there is provided a recombinant plasmid containing genetic material coding for a protein having L-asparaginase activity.

The genetic material contained in the plasmid may code for a protein which is identical to L-asparaginases produced by previously existing strains of L-asparaginase-producing bacteria, (hereinafter referred to as 'natural L-asparaginases') for example the Erwinia strains referred to previously, or the genetic material may code for proteins which differ from natural L-asparaginases in various ways. For example the genetic material may code for protein with or without a signal sequence (that is a sequence of generally hydrophobic amino acids at the N-terminus). Further the genetic material may code for protein which differs from natural L-asparaginase in variations in amino acid sequence or composition (provided that such variations do not result in any major adverse effect on the L-asparaginase activity of the enzyme).

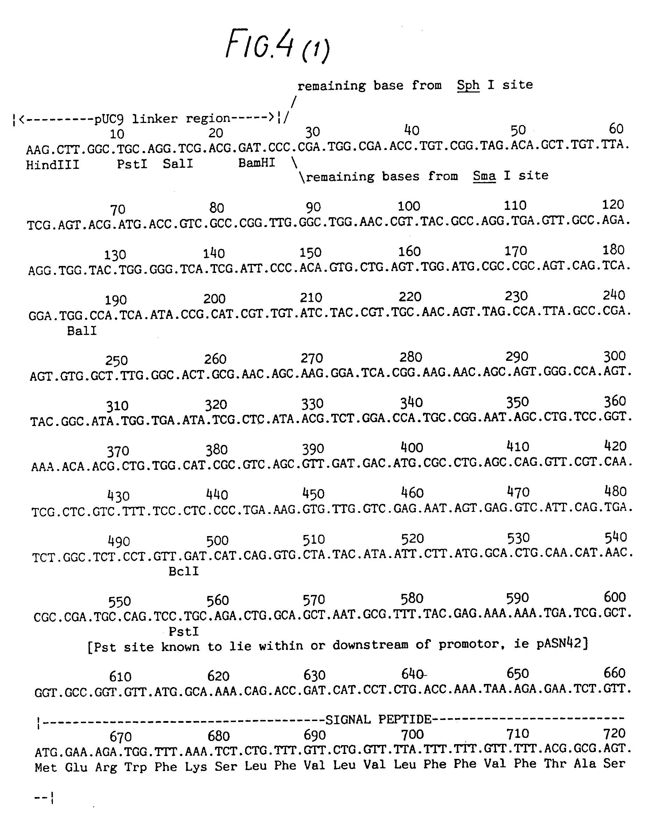

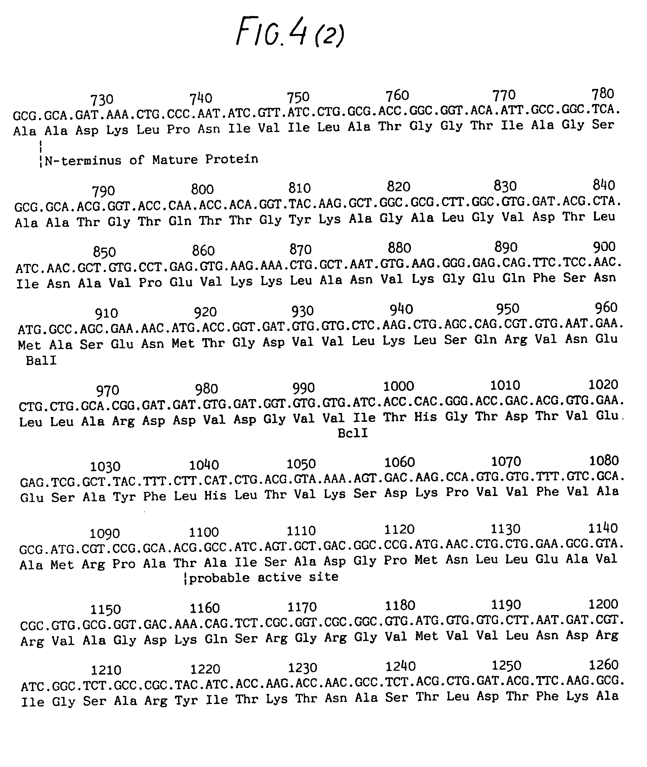

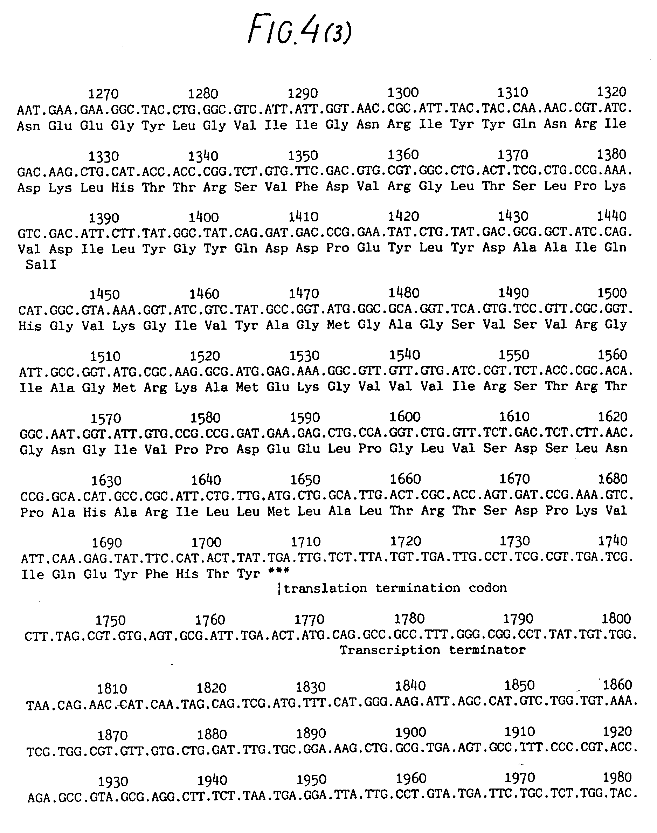

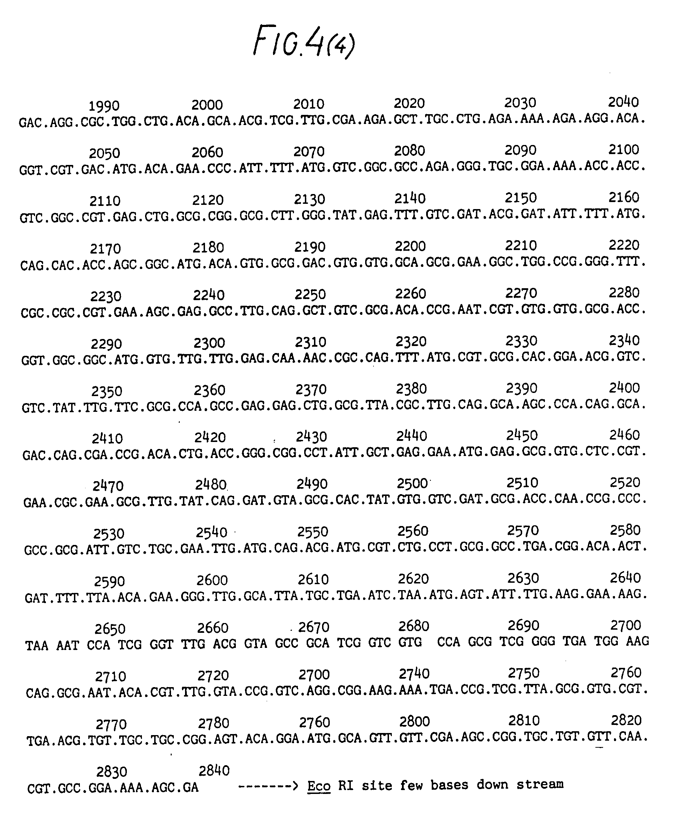

One particular exemplary class of plasmids according to the invention contain a DNA sequence identical to or related to and/or derived from the DNA sequence extending from the location marked 'N-Terminus' to the location marked 'Translation Termination Codon' of the DNA sequence shown in Figure 4. Where the DNA sequence is related to or derived from the DNA sequence shown in Figure 4, a number of possible relationships or derivation may be envisaged. Firstly as indicated above the DNA sequence may include additional bases, provided that the additional bases do not prevent expression of protein having L-asparaginase activity in a host organism. Thus for example the DNA sequence may include bases coding for a signal peptide, as in the sequence marked 'Signal Peptide' in Figure 4. Secondly, the DNA sequence may lack certain bases subject to the same constraint as indicated above. Thirdly, the DNA sequence may be related to or derived from the DNA sequence shown in Figure 4 by substituting bases for those shown. Generally such substitution should result in a DNA sequence which is capable of being expressed as protein having L-asparaginase activity, e.g. a sequence in which any triplet which is not identical to the corresponding triplet in the DNA sequence shown in Figure 4, nonetheless codes for the same amino acid

3

as the corresponding triplet.

The plasmids according to the invention preferably include a promotor which is operable in the selected species of micro-organisms or other host to be transformed by the plasmids to promote expression of L-asparaginase. A particularly preferred plasmid according to the invention is derived from plasmid pUC9 which contains the lac promotor and when introduced into a bacterial host reproduces with a high copy number.

Examples of hosts transformed with plasmids according to the invention include Escherichia coli and Erwinia chrysanthemi and Erwinia carotovora .

According to a further aspect of the invention there is provided a process for producing L-asparaginase which comprises culturing a host containing a plasmid as defined above and isolating L-asparaginase from the resulting culture.

L-asparaginase produced by expressing a recombinant gene coding for L-asparaginase and contained in a plasmid according to the invention forms a further aspect of the present invention.

The production of plasmids according to the invention and the production of L-asparaginase using such plasmids will now be described in more detail by way of example with particular reference to the accompanying Figures of which:

Figure 1 is schematic diagram illustrating the procedure whereby plasmid pASN326 was produced.

Figure 2 illustrates the production of plasmid pASN230.

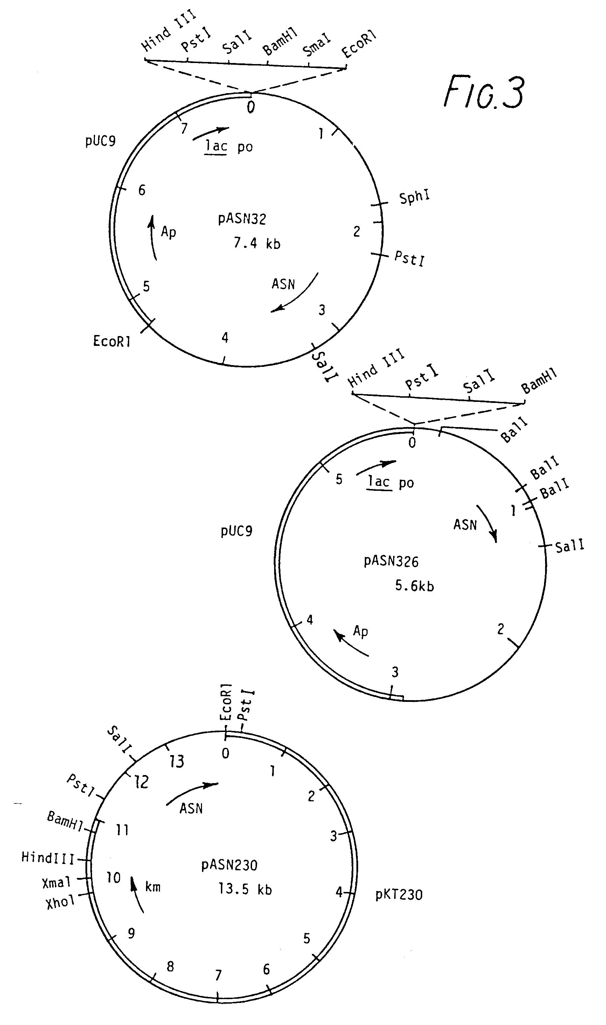

Figure 3 shows restriction maps of plasmids pASN32, pASN326 and pASN230.

Figure 4 is a nucleotide sequence and corresponding amino acid sequence of the DNA insert coding for L-asparaginase.

## GENERAL CLONING PROCEDURES

General procedures used to produce exemplary plasmids in accordance with the invention will now be described although it is to be appreciated that plasmids according to the invention may be produced by other techniques.

In order to produce plasmids containing genetic material coding for L-asparaginase, genomic DNA was isolated from an asparaginase-producing organism ( Erwinia chrysanthemi ), partially digested with the restriction enzyme Sau 3A and fragments of between 15 to 25-kb were isolated and cloned into the lambda vector 1059. Recombinant phage expressing E. chrysanthemi asparaginase were detected using purified anti-asparaginase IgG.

Four recombinant phage carrying the gene were shown to possess a common 4.7-kb Eco R1 DNA restriction fragment. This fragment was cloned into pUC9 in both orientations to generate the recombinant plasmids pASN30 and pASN32. A restriction map of the DNA insert was constructed and the position and orientation of the asparaginase structural gene determined by subcloning. The enzyme was produced at high levels in Escherichia coli (5% of soluble protein) and was shown to be exported to the periplasmic space. Purified asparaginase from E. coli cells carrying pASN30 was indistinguishable from the Erwinia enzyme on the basis of specific activity (660-700 U/mg), pI value (8.5), and subunit molecular weight (32 x $10^6$ ). Expression of the cloned gene was subject to glucose repression in E. coli but was not significantly repressed by glycerol.

As described in more detail below the asparaginase gene was present on a 4.7-kb Eco RI fragment and introduced into pUC9 in both directions, relative to the lac promotor of the vector, to give two plasmids designated pASN30 and pASN32. The asparaginase gene would theoretically be expected to be under the control of the lac promotor in the case of pASN32, but not pASN30. However it was found that the promotor of the gene functions perfectly well in E. coli (i.e. high levels of asparaginase were produced from pASN30) in its own right.

Taking this as a starting point we introduced the gene on a plasmid vector into the production strain. The pASN32 plasmid was chosen as there was the possibility that the lac promotor could contribute to aparaginase expression. An attempt was made to transform the production strain by published procedures, but this was unsuccessful. We next attempted to transform an Erwinia strain known to be transformable, Erwinia carotovora SCRI193. These experiments proved successful, and pASN32 was introduced into this strain.

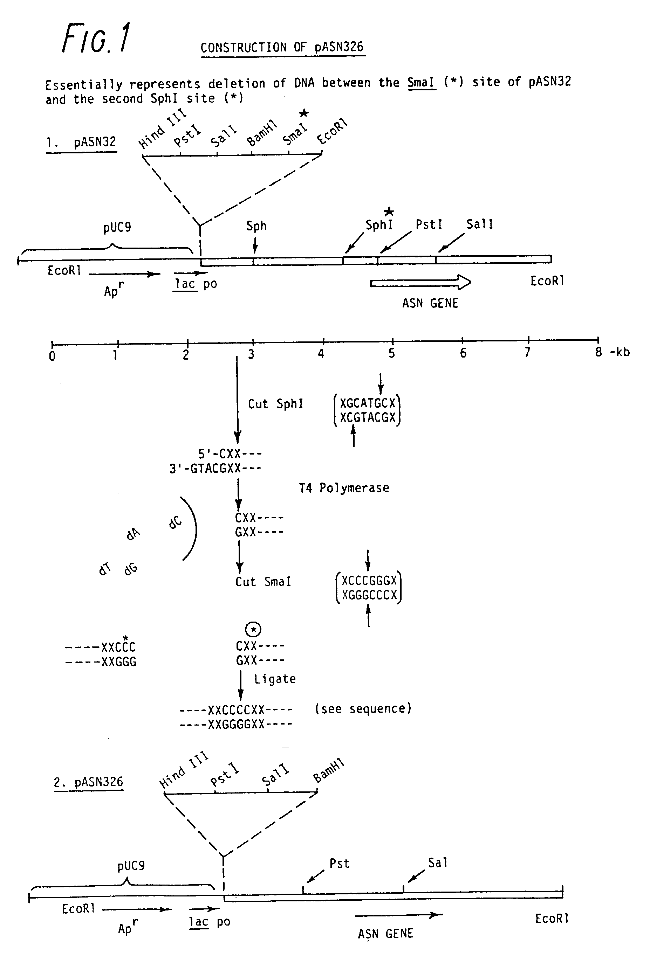

Attempts were also made to introduce the gene into other types of plasmid vector. In E. coli the plasmid pUC9 (based on the ColEI replicon) is present in very high copy number and is relatively stable, although this is not necessarily true in Erwinia . It was therefore decided to insert the gene into a vector known to replicate stably in a wide range of Gram -ve bacteria. We chose the E. coli / Pseudomonas shuttle vector pKT230 (based on the replicon of RSF1010). Due to the availability of restriction sites in this

4

particular vector a certain amount of genetic engineering was necessary as will be described below and illustrated in Figure 1.

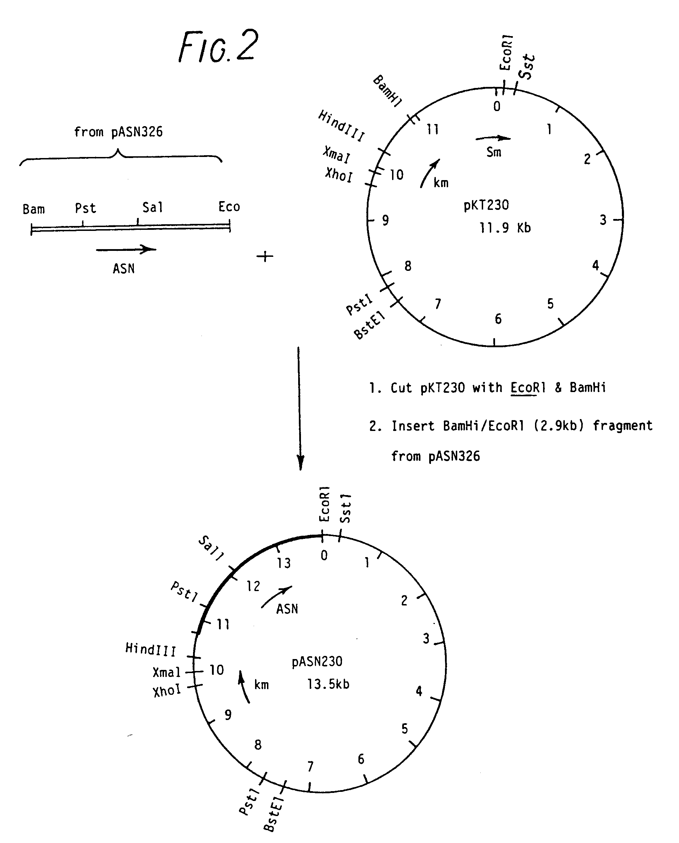

The plasmid pASN32 was cleaved with SphI and the DNA blunt-ended by treatment with T4 polymerase. The plasmid was then cleaved with the blunt-end enzyme Sma I and religated to yield the plasmid pASN326. The essential difference between pASN32 and pASN326 is that the latter has had a tract of DNA deleted between the second Sph I site and the Sma I site. Due to the nature of this construction the Sph I and Sma I sites of the plasmid have been lost. Thus the asparaginase gene can be excised from pASN326 by cleavage with Bam HI and Eco RI. This fragment was inserted into the plasmid pKT230, at its complementary Bam HI and Eco RI sites, to yield the plasmid pASN230 (see Fig. 2). The insertion of the gene in pKT230 is such that the promotor of the vector's Km resistance gene could, in theory, effect expression of the asparaginase gene.

The above recombinant plasmids containing the asparaginase gene were used to transform other microorganisms, particularly Erwinia chrysanthemi B374 and Pseudomonas putida KT 2440*. The transformed strains produced high levels of asparaginase.

In the following description reference will be made to the following general procedures:

## MICROBIAL STRAINS AND PLASMIDS

Microbial strains and plasmids used in accordance with the invention are listed in Table 1. Bacteriophage $\lambda$ was grown in E. coli Q358 or Q359 as described by Maniatis et al. (1982).

## MEDIA

E. coli strains were cultured in L-broth (1% tryptone, 0.5% yeast extract, 0.5% NaCl). Media were solidified with either the addition of 2% (w/v) Bacto-agar (Difco) or 0.8% agarose (Sigma). Glucose or glycerol, when required, were added to media at a final concentration of 2 mg ml$^{-1}$. Ampicillin (100 $\mu$g ml$^{-1}$) was used for the selection and growth of transformants. Functional $\beta$-galactosidae was detected by the addition of 5-bromo-4-chloro-indoyl-$\beta$-D-galactoside (X-Gal) to a final concentration of 2 $\mu$g ml$^{-1}$.

## CHEMICALS

Na$^{125}$I (carrier free; 100 m Ci ml$^{-1}$) and the in vitro packaging kit were obtained from Amersham International. Agarose, restriction enzymes and T4 DNA ligase were purchased from Bethesda Research Laboratory. Formamide was from Fluberg. PVC discs were obtained from A. Tweed (Salisbury UK) O-OMAT RP x-ray film was purchased from Kodak. CNBr activated Sepharose 4B was obtained from Pharmacia. All other reagents were obtained from Sigma Chemical Co. or BDH.

## DNA ISOLATION

(i) E. chrysanthemi DNA

Mid-log cells of E. chrysanthemi were harvested and lysed by the 'Brij Lysis' method (Clewell and Helinski, 1969). DNA was extracted as described by Gilbert et al. (1985). The final DNA preparation was severely degraded. This was similar to the observation of Ween et al. (1984).

(ii) Plasmid DNA

E.coli plasmids were purified from 1 litre of L-broth cultures containing ampicillin by 'Brij Lysis' and subsequent CsCl density centrifugation (Clewell and Helinski, 1969). The rapid boiling method of Holmes and Quigley (1981) was employed for small scale plasmid isolation for screening purposes.

## CELL FRACTIONATION TECHNIQUES

The cytoplasmic, membrane and periplasmic fractions of E. coli were prepared as described by Minton et al. (1983).

* "Efficient Transformation of Erwinia carotovora Subsp. carotovora and E. carotovora Subsp. atroseptica" JCD Hinton, MCH Perombelon, GPC Salmond; J. Bact. Vol. 161, pp. 341-343 (1985).

ASSAY OF ASPARAGINASE

Asparaginase catalytic activity was determined by the method of Wade and Phillips (1971).

1. Construction of phage library of E.chrysanthemi DNA

A phage library of E. chrysanthemi DNA was constructed as described by Karn et al. (1980). Briefly, E. chrysanthemi DNA was partially digested with Sau3a and DNA fragments 15-25 kilobase-pair (kb) in size were purified by agarose gel electrophoresis. The restriction fragments were ligated to 1059 DNA cleaved with Bam Hi. The DNA was in vitro packaged, and the spi −phenotype of recombinant phage was selected by growth on the E. coli P2 lysogen Q350.

2. Production of anti-asparaginase

3 mg of purified asparginase in incomplete Freund's adjuvant was injected into a rabbit on day 1. A repeat dose was given on day 14 and the rabbit bled on day 28. Red blood cells are removed by centrifugation and the IgG fraction of the serum obtained by $(NH_4)_2SO_4$ precipitation.

3. Purification of anti-asparaginase IgG

Purified asparaginase was immobilised on CNBr-activated Sepharose 4B according to the manufacturer's instructions, at a concentration of 2 mg of protein per ml of gel. The IgG containing $(NH_4)_2SO_4$ fraction of asparaginase antiserum was passed over the column and unbound protein removed by washing with 20 mM/sodium phosphate buffer (pH 7.5), 0.15/NaCl. Anti-asparaginase IgG was eluted with 0.1M-glycine, HCl (pH 3.0), 0.5M-NaCl. The pH was immediately adjusted to pH 7.0 with 1M-Tris/HCl pH 5.0 and the antibody was stored at 4°C in the presence of 0.01% $NaN_3$.

Isolation of the asparaginase gene

A clone bank of E. chrysanthemi DNA was constructed in bacteriophage λ 1059 and a total of 20,000 recombinants isolated. The library was screened for phage which synthesised asparaginase antigen

5. Screening of the λ library for asparaginase gene

For screening the λ library, recombinant phage were grown on E. coli strain Q358 to a density of 100 plaques per $cm^2$. Plaques were screened for the presence of asparaginase antigen by the method of Broome and Gilbert (1979). The IgG fraction of asparaginase antiserum was used to coat the PVC discs. Purified $^{125}$I-labelled anti-asparaginase IgG was used as the radiolabelled probe. (When an attempt was made to use the IgG fraction of asparaginase antiserum as the radioactive probe, very severe background problems resulted.)

More specifically, the phage library, containing E. chrysanthemi DNA, was grown on E. coli strain Q358. Agar plates containing recombinant plaques (each $100\ cm^2$ plate had $10^3$ plaques) were incubated with PVC discs coated with anti- Erwinai asparaginase antiserum for 3 hours at 4°C. The discs were removed, thoroughly washed with ice-cold distilled water to remove cell debris, and then incubated with 20 mM-sodium phosphate buffer (pH 7.4) containing 0.15M-NaCl, 0.5% (w/v) bovine serum albumin and $^{125}$I-labelled anti- Erwinia asparaginase ($3 \times 10^7$ cpm $ug^{-1}$IgG; $4 \times 10^6$ cpm $ml^{-1}$) for 16 hours at 4°C (1.5 ml of buffer per disc). The discs were then washed 4 times in 20 volumes of 20 mM-sodium phsophate buffer (pH 7.4) containing 0.15 M-NaCl, 0.5% bovine serum albumin, two times in distilled water and dried at room temperature. The discs were then exposed to x-ray film with an intensifying screen at -70°C for 48 hours. Plaques containing Erwinia asparaginase antigen produce black dots on the film.

Approximately 20 phage were shown to produce antigen, indicating that these recombinants carried at least part of the asparaginase gene.

6. General recombinant DNA procedures

DNA was extracted from four of these phage and digested with various restriction enzymes. A 4.7-kb Eco RI DNA fragment, common to all four phage, was subcloned into the Eco RI site of pUC9 in both orientations to yield the recombinant plasmids pASN30 and pASN32. Cells of E. coli JM83 harbouring either plasmid were shown to synthesise high levels of both catalytically and antigenically active asparaginase (Table 2). A restriction enzyme map of the E. chrysanthemi DNA inserted in pASN30 is shown in Figure 2.

Restriction endonucleases and T4 DNA ligase were used according to the manufacturers instructions. Transformation of E. coli was performed as described by Cohen et al. (1972). DNA was electrophoresed in agarose gels (0.8-1.5%) using a Tris-borate-EDA buffer (Meyers et al., 1976). DNA digested with Hind III or Hind III/ Eco RI and 0 X174 RF digested with Hind III were used as mol of standards. All recombinant DNA work was done under Category I containment.

7. Radioimmunoassay of Asparaginase

(i) Preparation of polyvinyl chloride (PVC) discs coated with Erwinia asparaginase antiserum

PVC discs (120 mm$^2$) obtained from H. Tweed (Salisbury, UK) were incubated for 5 minutes at room temperature with 200 ml of 0.1 M-NaHCO$_3$/NaOH buffer (pH 9.2) containing 60 $\mu$g ml$^{-1}$ of Erwinia asparaginase antiserum. The discs were then washed in 20 mM-sodium phosphate buffer (pH 7.4) containing 0.15 M-NaCl and 0.5% (w/v) bovine serum albumin, rinsed in distilled water and dried at room temperature.

(ii) Preparation of anti-Erwinia asparaginase IgG

Purified Erwinia asparaginase was immobilised on CNBr-activated Sepharose 4B, according to the manufacturer's instructions, at a concentration of 2 mg of protein ml$^{-1}$ of gel. IgG containing (NH$_4$)$_2$SO$_4$ fraction of Erwinia asparaginase antiserum was passed over the column and unbound protein removed by washing with 20 mM-sodium phosphate buffer pH 7.4, containing 0.15 M-NaCl. Anti- Erwinia asparaginase IgG was eluted with 0.1M-glycine/HCl buffer (pH 3.0), containing 0.5 M-NaCl . The pH was immediately adjusted to pH 7.0 with 1M-Tris/HCl buffer pH 8.0, and the antibody was stored at 4°C in the presence of 0.01% NaN$_3$.

(iii) Labelling anti-Erwinia asparaginase IgG

The antibody was labelled with I$^{125}$ as follows. The 100 $\mu$l reaction mixture containing 0.1 M-potassium phosphate buffer (pH 7.2) 1mCi of carrier-free Na$^{125}$I, 20 ug of anti- Erwinia asparaginase IgG and 2 $\mu$g of chloramine T was incubated for 1 minute at room temperature. Potassium iodide was added to a 100-fold excess and the $^{125}$I-labelled IgG purified on a Sephadex GSO column equilibrated with PBS + 0.5% bovine serum albumin. The specific activity of radiolabelled IgG was 3 x 10$^7$ cpm $\mu$g$^{-1}$ of IgG.

All components in the assay were diluted with 50 mM-potassium phosphate buffer, pH 7.4 containing 0.85% NaCl acid 0.1% bovine serum albumin. EA antiserum, 0.1 ml of a 10$^5$-fold dilution, was mixed with 0.1 ml a EA standard solution, 1 $\mu$g-10 ng/ml or a dilution of the test sample. Finally 0.1 ml of $^{125}$I-labelled EA (2 x 10$^5$ c.p.m./ml) was added and tubes incubated overnight at 20°C. Donkey anti-rabbit IgG, 0.1 ml of a 50-fold dilution of serum supplied by Wellcome Reagents Ltd (Beckenham, Kent, U.K.) and 0.1 ml of a 300-fold dilution of normal rabbit serum were then added and the tubes incubated for 3 hours at room temperature. After adding 1 ml of water, the tubes were centrifuged for 30 minutes at 45 x 10$^3$ g. The supernatants were aspirated and the tubes counted in a LICB 1270 Rackgamana counter. Blanks consisted of all components of the assay except EA. Approximately 30% of the $^{125}$I-labelled EA bound to its antibody in the absence of unlabelled enzyme.

8. Subcloning and Orientation of the asparaginase gene

The position of the asparaginase gene within the 4.7-kb Eco RI DNA fragment was determined by deleting and subcloning various restriction fragments into pUC8 or pUC9, and determining asparaginase activity in the resultant recombinant clones. The asparaginase gene in pASN30 was still active after deletion of the 1.3-kb Sph I fragment (Fig. 2), but was inactivated by deletion of either the 5.95-kb Sal I or 5.1-kb Pst I fragment. When the Pst I(5.1)- Eco RI(7.4) fragment was cloned into pUC8, cells carrying the resultant recombinant plasmid (pASN40) did not produce detectable levels of asparaginase. In contrast, cells harbouring a recombinant plasmid (pASN42) generated by inserting the same fragment into pUC9 were found to produce the enzyme. This implies that the expression of the asparaginase gene from the Eco RI-Pst I fragment is dependant on a extraneous promoter, presumeably the lac promoter which lies upstream of the polylinker region of both pUC8 and pUC9. To test this possibility the compatible plasmid pNM52 (pACYC184 carrying the lac Iᑫ gene) was introduced into JM83 carrying either pASN40 or pASN42.

The plasmid pNM52 is a multicopy plasmid carrying the lac Iᑫ gene, which is compatible with vectors utilising a ColEI replicon. A detailed description of the construction of this plasmid will be described elsewhere. Essentially a 9-kb Eco RI restriction fragment carrying the lac I¹ gene was subcloned from the plasmid pHIQ6 (Hare and Sadler, 1978) into the Eco RI site of pACYC184 (Change and Cohen, 1978). The Eco RI insert carrying lacI ᵃ was then reduced in size by deleting a 4.4-kb Pst1 DNA fragment to yield pNM52. The plasmid carries DNA from the E. coli lac operon extending from approximately 0.4-kb upstream of the lac Iᑫ gene through to the Eco RI site at the C-Terminus of the lac Z gene (Kalnins et al., 1983).

In the case of cells carrying pNM52 and pASN42, detectable levels of asparaginase were only observed following induction of the lac promoter with isopropyl-thioβ-galacosidase (IPTG). Addition of IPTG to cells harbouring pNM52 and pASN40 did not elicit asparaginase production.

The above data localise the asparaginase structural gene to a region between the Pst I and Eco RI restriction sites at map positions 5.1 and 7.4 respectively (Fig. 2). The orientation dependant expression of asparaginase from the 2.3-kb Pst I- Eco RI fragment, compared to the orientation independant expression of the 4.7-kb Eco RI fragment, indicates that Pst I cleaves either within or downstream of the asparaginase promoter region. Furthermore, regulatory control of asparaginase expression by the lac promoter of pASN42 allows the deduction of the direction of transcription of the gene. Thus, in pASN30 (Fig. 2) the asparaginase gene is transcribed in the opposite orientation to the β-lactamase and lac Z' genes.

## 9. Expression of the cloned gene in E. coli

Cells harbouring pASN30 were shown to produce approximately 6% of soluble protein as asparaginase. To determine whether the cloned product in E. coli has the same properties as enzyme from E. chrysanthemi , the asparaginase was purified from both enterobacteria and characterised. A specific activity of 660 U mg of protein was obtained for asparaginase purified from E. coli , which compares favorably with the value 700 U mg of protein observed with the enzyme isolated from Erwinia . Asparaginase isolated from both enterobacteria had an identical pI value (8.5) and sunbunit molecular weight ($32 \times 10^3$), and in both cases appeared to be tetrameric as determined by gel filtration.

## 10. Periplasmic localisation of asparaginase

Previous work has demonstrated that asparaginase is located in the periplasm of Erwinia chrysanthemi - (Wade personal communication). The localisation of the cloned enzyme in E. coli was therefore examined by the separation of cellular proteins into cytoplasmic, periplasmic and whole membrane fractions. As a control, the levels of three marker enzymes, alkaline phosphatase (periplasmic), glyceraldehyde 3-phosphate dehydrogenase (cytoplasmic), and NADH.O oxidoreductase (membrane bound), were also determined. Asparaginase was found to occur predominantly (89%) in the periplasm.

## 11. Regulation of the cloned asparaginase gene in E. coli

Production of asparaginase in E. chrysanthemi is known to be repressed by both glucose and glycerol (Callow et al., 1971). The regulation of the cloned gene was therefore investigated using E. coli JM83 containing the plasmid pASN30. Cells were cultured in L-broth with and without glucose or glycerol, and the level of asparaginase expression monitered. Enzyme production was markedly repressed in the presence of glucose, but only slightly inhibited by glycerol.

It can be seen from the procedures and results described above that the E. chrsyanthemi gene coding for L-asparaginase has successfully been cloned and expressed in E. coli . Initially the gene was localised to 4.7-kb Eco RI restriction fragment, which was cloned into pUC9 in both orientations to give the recombinant plasmids pASN30 and pASN32. E. coli cells harbouring either plasmid produced similar levels of asparaginase, indicating that the promoter region of the gene had also been clotted. The high level of expression (up to 6% soluble protein) also suggests that this particular Erwinia promoter is efficiently utilised in E. coli . The promoter region of an E. chrysanthemi pectate lyase gene has also been shown to be sufficiently utilised in E. coli (Keen et al., 1984). Secretion of asparaginase by E. coli was also demonstrated as the mature enzyme was predominantly located in the periplasmic space. It follows that the asparaginase structural gene should contain an N-terminal signal peptide sequence. This has been confirmed by comparing the base sequence of the gene with the N-terminal sequence of mature L-asparaginase (Fig. 4).

8

Further subcloning demonstrated that the asparaginase structural gene was contained within a 2.3-kb Pst I/ Eco RI restriction fragment. Recombinant plasmids, constructed by inserting this fragment into either pUC8 (pASN40) or pUC9 (pASN42) indicated that the expression of the asparaginase gene was dependant upon the orientation of the Erwinia DNA relative to the lac promoter of the pUC vectors. Thus JM83 ( lac I) cells carrying pASN40 did not produce detectable levels of asparaginase, whereas cells harbouring pASN42 gave high levels of the enzyme. The introduction of the lac I$^q$ gene into JM83 cells carrying pASN42 demonstrated that asparaginase expression was totally dependant on the vector's lac promoter, as the appearance of the enzyme was only elicited by the addition of the lac inducer, IPTG. These results indicate that the functional asparaginase promoter present on the 4.7-kb Eco RI fragment has been inactivated on the 2.3-kb Pst I/ Eco RI sub-fragment. The Pst I site must therefore lie either within or downstream of the Erwinia promoter. It follows that the direction of transcription of the asparaginase gene must proceed from the Pst I to the Eco RI end of the 2.3-kb Erwinia DNA fragment.

The level of expression of the asparaginase gene when under lac promoter control (pASN42) was lower than when enzyme synthesis was regulated by the natural asparaginase promoter (pASN30). This was surprising as the lac promoter is widely recognised as one of the more powerful E. coli promoters (Rosenberg et al., 1982). Two possible explanations may be: (i) the mRNA produced from the lac promoter is inefficiently translated, possibly due to secondary structure (e.g. Schoner et al., 1984), or (ii) fusion of the N-terminal portion of the lac Z' gene with the asparaginase structural gene results in a decreased enzymic activity. The latter possibility would appear unlikely as the protein produced by E. coli cells carrying pASN42 was localised exclusively in the periplasmic space indicating that the protein synthesised still carried an N-terminal signal sequence.

Expression of the cloned gene was shown to be subject to catabolite repression in E. coli . As asparaginase production in E. chrysanthemi is also glucose repressed, the regulatory region of the gene must contain a recognition sequence for the catabolite activator protein. Furthermore, this recognition sequence must be conserved in both enterobacteria. A similar situation was observed with the cloned pectate lyase gene (Keen et al., 1984). In contrast to the regulatory control of asparaginase synthesis in Erwinia , expression of the cloned gene was not repressed by glycerol in E. coli . This implies that the mechanism of glycerol and glucose repression of asparaginase are distinct.

## 12. Comparison of level of production of L-asparaginase

The levels of production of L-asparaginase by transformed hosts in accordance with the invention ( Ewinia carotovora SCRI 193 containing plasmids pASN32, pASN326 or pASN230) were compared with those of (a) the untransformed host and (b) a currently used production strain, Erwinia chrysanthemi NCPB 1066.

500 mls of E. coli harbouring pASN32 was grown overnight in yeatex and glutamate supplemented with ampicillin (100 ug ml$^{-1}$). Cells were harvested $10^4$ x g for 5 minutes at 4°C and resuspended in 10 mls of 50mM-Tris. The bacteria were disrupted by sonication and cell debris removed by centrifugation at 16 x $10^3$ g for 10 minutes at 4°C. The cell-free extract was equilibrated in 20 mM-sodium phosphate buffer (pH 6.3) by gel filtration. The extract was then applied to a CM-cellulose column (10 ml bed volume) equilibrated in the same phosphate buffer. Unbound protein was washed through the column, and bound protein was eluted with a 100 ml linear sodium phosphate gradient (20 mM- 80mM, pH 6.3). Asparaginase was eluted in a single sharp peak at a phosphate concentration of 40 mM. The enzyme preparation was homogenous as judged by sodium dodecyl sulphate polyacrylamide gel electrophoresis and of high specific activity (660 U mg$^{-1}$ of protein).

The following asparaginase levels were detected in the culture media:

| STRAIN | UNITS PER ML CULTURE | SPECIFIC ACTIVITY (UNITS PER MG PROTEIN) |
|---|---|---|
| Erwinia carotovora SCRI 193 | 1.02 | 1.81 |
| SCRI 193 + pASN32 | 11.19 | 53.34 |
| SCRI 193 + pASN326 | 8.98 | 39.41 |
| SCRI 193 + pASN230 | 20.84 | 42.9 |
| Erwinia chrysanthemi NCPPB 1066 | 10.87 | 14.97 |

It can be seen from the above results that the specific activity of product produced by each of the transformed host micro-organisms according to the invention was higher than in the controls and two of the micro-organisms produced L-asparaginase in a higher yield than the production strain NCPB 1066.

Cultures of microorganisms containing plasmids referred to herein have been deposited with National Collection of Industrial Bacteria on 5th August 1985 under the following serial numbers:

| E. Coli | MC1061 | pASN 326 | NCIB 12129 |
| E. Coli | MC1061 | pASN 230 | NCIB 12130 |
| E. Coli | JM83 | pASN 30 | NCIB 12131 |
| E. Coli | JM83 | pASN 32 | NCIB 12132 |
| E. Coli | JM83 | pASN 42 | NCIB 12133 |

## Table 1 - Microbial Strains And Plamids Used In This Work

| Strain Plasmid or Genotype or Bacteriophage Phenotype | | Source or Reference |
|---|---|---|
| E. chrysanthemi NCPPB 1066 | | PHLS, Porton Down |
| E. chrysanthemi B374 | | Hamon, Y; Peron, Y (1961) CR Acad. Sci. Vol. 253, pp. 913-15 |
| E. carotovera SCRI 193 | | Hinton et al. (supra) J. Bact. Vol. 161, pp. 341-43 (1965) |
| P. putida KT2440 | | Bagdasarian, M; Lurz, R; Rurzkert, B; Franklin, M.M; Bagdasarian, M; Frey, J; Timmis, K.N; Gene (1981) Vol. 116, pp. 237-47 |
| E. coli | | |
| Q358 | hsdR $80^r$ SupE | Karn et al. (1980) |
| Q359 | hsdR $80^r$ SupE P2 | Karn et al. (1980) |
| JM83 | ara $\triangle$ (lac-pro) rps L thi $\emptyset$ 80d lacI$^r$ZM15 | Vieira & Messing (1982) |
| Bacteriophage | | |
| 1059 | | Karn et al. (1980) |
| Plasmids | | |
| pUC9 | Amp$^R$lac$^{Z'}$ | Vieira & Messing (1982) |
| pUC8 | Amp$^R$lac$^{Z'}$ | "       "       " |
| pASN30 | Amp$^R$ expresses EA | Novel Strains |
| pASN32 | Amp$^R$ expresses EA | "       " |
| pASN42 | Amp$^R$ expresses EA under control of lacP | "       " |
| pASN37 | Amp$^R$ no EA activity | "       " |
| pASN52 | lac I$^q$tet$^R$ | "       " |

## Claims

1. A process for producing L-asparaginase which comprises culturing an L-asparaginase-producing microorganism, characterised in that the microorganism is a transformed host microorganism containing a recombinant plasmid containing genetic material coding for a protein having L-asparaginase activity and comprising at least a portion of the DNA sequence extending from the location marked 'N-terminus' to the location marked 'Translation Stop Codon' of the sequence shown in Figure 3 or a sequence related to or derived from that sequence and capable of being expressed as protein having L-asparaginase activities, whereby said protein is produced at a level of at least 5% of soluble protein.

2. A process according to Claim 1 wherein the genetic material is derived from DNA from the genome of an Erwinia species.

3. A process according to Claim 2 wherein the Erwinia species is Erwinia carotovora.

4. A process according to any preceding claim wherein said recombinant plasmid is selected from the recombinant plasmids designated pASN32 (NCIB 12132), pASN326 (NCIB 12129), and pASN230 (NCIB 12130).

5. A process according to any preceding claim wherein said transformed host is derived from a strain of Escherichia coli or Erwinia chrysanthemi.

6. A process according to Claim 5 wherein said transformed host is derived from E. chrysanthemi NCPPB 1066.

7. A process according to Claim 5 wherein said transformed host is derived from E. chrysanthemi B 374.

8. A process according to any of Claims 1 to 4 wherein said transformed host is derived from E. carotovora.

9. A process according to Claim 8 wherein said transformed host is derived from E. carotovera SCRI 193.

10. A process according to any of Claims 1 to 4 wherein said transformed host is derived from a strain of Pseudomonas putida.

11. A process according to Claim 10 wherein said transformed host is derived from P. putida KT 2440.

12. A recombinant plasmid containing at least a portion of the DNA sequence extending from the location marked 'N-terminus' to the location marked 'Translation Stop Codon' of the sequence shown in Figure 3 or a sequence related to or derived from that sequence and capable of being expressed as protein having L-asparaginase activities.

13. The recombinant plasmids designated pASN32 (NCIB 12132), pASN326 (NCIB 12129) and pASN230 (NCIB 12130).

14. A host transformed with a recombinant plasmid according to Claim 12 or Claim 13.

15. A transformed host according to Claim 14 derived from a strain of Escherichia coli or Erwinia chrysanthemi.

16. A transformed host according to Claim 15 derived from E. chrysanthemi NCPPB 1066, E. chrysanthemi B 374 or E. carotovera.

17. A transformed host according to Claim 16 derived from E. carotovera SCRI 193.

18. A transformed host according to Claim 14 derived from a strain of Pseudomonas putida.

19. A transformed host according to Claim 18 derived from P. putida KT 2440.

**Patentansprüche**

1. Verfahren zur Herstellung von L-Asparaginase, bei dem man einen L-Asparaginase produzierenden Mikroorganismus kultiviert, dadurch gekennzeichnet, daß der Mikroorganismus ein transformierter Wirtsmikroorganismus ist, der ein rekombinantes Plasmid enthält, das genetisches Material enthält, das für ein Protein mit L-Aspariginaseaktivität codiert, und mindestens einen Teil der DNA-Sequenz, die von der mit "N-Terminus" bezeichneten Stelle bis zu der mit "Translation Stop Codon" bezeichneten Stelle der in Fig. 3 gezeigten Sequenz reicht, oder eine damit verwandte oder davon abgeleitete Sequenz enthält und in der Lage ist, als Protein mit L-Asparaginase-Aktivitäten exprimiert zu werden, wodurch das Protein in einer Konzentration von mindestens 5 % des löslichen Proteins gebildet wird.

2. Verfahren nach Anspruch 1, bei dem das genetische Material aus der DNA des Genoms einer *Erwinia*–Spezies stammt.

3. Verfahren nach Anspruch 2, bei dem die *Erwinia*-Spezies *Erwinia carotovora* ist.

4. Verfahren nach einem der vorstehenden Ansprüche, bei dem das rekombinante Plasmid aus den pASN32 (NCIB 12132), pASN326 (NCIB 12129) und pASN230 (NCIB 12130) bezeichneten rekombinierten Plasmiden ausgewählt wird.

5. Verfahren nach einem der vorstehenden Ansprüche, bei dem der transformierte Wirt aus einem Stamm *Escherichia coli* oder *Erwinia chrysanthemi* abgeleitet ist.

6. Verfahren nach Anspurch 5, bei dem der transformierte Wirt von *E. chrysanthemi* NCPPB 1066 abgeleitet ist.

7. Verfahren nach Anspruch 5, bei dem der transformierte Wirt von *E. chrysanthemi* B 374 abgeleitet ist.

8. Verfahren nach Anspruch 1 bis 4, bei dem der transformierte Wirt von *E. carotovora* abgeleitet ist.

9. Verfahren nach Anspruch 8, bei dem der transformierte Wirt von *E. carotovora* SCRI 193 abgeleitet ist.

10. Verfahren nach einem der Ansprüche 1 bis 4, bei dem der transformierte Wirt von einem *Pseudomo*–*nas putida*-Stamm abgeleitet ist.

11. Verfahren nach Anspruch 10, bei dem der transformierte Wirt von *P. putida* KT 2440 abgeleitet ist.

12. Rekombinantes Plasmid, das mindestens einen Teil der DNA-Sequenz, die von der mit "N-terminus" bezeichneten Stelle zu der mit "Translation Stop Codon" bezeichneten Stelle der in Figur 3 gezeigten Sequenz reicht, oder eine damit verwandte oder davon abgeleitete Sequenz enthält und in der Lage ist, als Protein mit L-Asparaginase-Aktivitäten exprimiert zu werden.

13. Die als pASN32 (NCIB 12132), pASN326 (NCIB 12129) und pASN230 (NCIB 12130) bezeichneten rekombinanten Plasmide.

14. Transformierter Wirt, der mit einem rekombinanten Plasmid nach Anspruch 12 oder 13 transformiert ist.

15. Transformierter Wirt nach Anspruch 14, der aus einem Stamm *Escherichia coli* oder *Erwinia chrysanthemi* abgeleitet ist.

16. Transformierter Wirt nach Anspruch 15, der aus *E. chrysanthemi* NCPPB 1066, *E. chrysanthemi* B 374 oder *E. carotovora* abgeleitet ist.

17. Transformierter Wirt nach Anspruch 16, der aus *E. carotovora* SCRI 193 abgeleitet ist.

18. Transformierter Wirt nach Anspruch 14, der aus einem Stamm *Pseudomonas putida* abgeleitet ist.

19. Transformierter Wirt nach Anspruch 18, der aus *P. putida* KT 2440 abgeleitet ist.

# EP 0 211 639 B1

**Revendications**

1. Procédé pour la production de L-asparaginase qui comprend la culture d'un microorganisme produisant de la L-asparaginase, caractérisé en ce que le microorganisme est un microorganisme hôte transformé contenant un plasmide recombinant contenant le matériel génétique codant pour une protéine ayant une activité L-asparaginase et comprenant au moins une partie de la séquence d'ADN s'étendant du locus marqué "N-terminal" au locus marqué "Codon stop de traduction" de la séquence montrée dans la figure 4 ou une séquence apparentée ou dérivée de cette séquence et capable d'être exprimée en tant que protéine ayant une activité L-asparaginase, de telle sorte que cette protéine est produite à raison d'au moins 5% de protéine soluble

2. Procédé suivant la revendication 1, dans lequel le matériel génétique dérive de l'ADN du génome d'une espèce Erwinia.

3. Procédé suivant la revendication 2, dans lequel l'espèce Erwinia est Erwinia carotovora.

4. Procédé suivant l'une quelconque des revendications précédentes, dans lequel ce plasmide recombinant est choisi parmi les plasmides recombinants désignés pASN32 (NCIB 12132, pASN326 (NCIB 12129) et pASN230 (NCIB 12130).

5. Procédé suivant l'une quelconque des revendications précédentes, dans lequel cet hôte transformé dérive d'une souche d'Escherichia coli ou d'Erwinia chrysanthemi.

6. Procédé suivant la revendication 5, dans lequel cet hôte transformé dérive de E. chrysanthemi NCPPB 1066.

7. Procédé suivant la revendication 5, dans lequel cet hôte transformé dérive de E. chrysanthemi B 374.

8. Procédé suivant l'une quelconque des revendications 1 à 4, dans lequel cet hôte transformé dérive d'E. carotovora.

9. Procédé suivant la revendication 8, dans lequel cet hôte transformé dérive d'E. carotovora SCRI 193.

10. Procédé suivant l'une quelconque des revendications 1 à 4, dans lequel cet hôte transformé dérive d'une souche de Pseudomonas putida.

11. Procédé suivant la revendication 10, dans lequel cet hôte transformé dérive de P. putida KT 2440.

12. Plasmide recombinant contenant au moins une partie de la séquence d'ADN s'étendant du locus marqué "N-terminal" au locus marqué "Codon stop de traduction" de la séquence montrée dans la figure 4 ou une séquence apparentée ou dérivée de cette séquence et capable d'être exprimée en tant que protéine ayant une activité L-asparaginase.

13. Plasmides recombinants désignés pASN32 (NCIB 12132, pASN326 (NCIB 12129) et pASN230 (NCIB 12130).

14. Hôte transformé avec un plasmide recombinant suivant les revendications 12 ou 13.

15. Hôte transformé suivant la revendication 14, dérivé d'une souche d'Escherichia coli ou d'Erwinia chrysanthemi.

16. Hôte transformé suivant la revendication 15, dérivé d'E. chrysanthemi NCPPB 1066, E. chrysanthemi B 374 ou E. carotovora.

17. Hôte transformé suivant la revendication 16, dérivé d'E. carotovora SCRI 193.

18. Hôte transformé suivant la revendication 14, dérivé d'une souche de Pseudomonas putida.

14

**19.** Hôte transformé suivant la revendication 18, dérivé de P. putida KT 2440.

# FIG. 1

CONSTRUCTION OF pASN326

Essentially represents deletion of DNA between the SmaI (*) site of pASN32 and the second SphI site (*)

Cut SphI

5'-CXX---
3'-GTACGXX---

$\left(\begin{array}{c} \text{XGCATGCX} \\ \text{XCGTACGX} \end{array}\right)$

T4 Polymerase

CXX----
GXX----

dA    dC
dT    dG

Cut SmaI

$\left(\begin{array}{c} \text{XCCCGGGX} \\ \text{XGGGCCCX} \end{array}\right)$

----XXCCC
----XXGGG

⊛
CXX----
GXX----

Ligate

----XXCCCCXX----    (see sequence)
----XXGGGGXX----

16

# FIG.2

from pASN326

1. Cut pKT230 with EcoR1 & BamHi

2. Insert BamHi/EcoR1 (2.9kb) fragment from pASN326

FIG.3

# FIG.4 (1)

```
                                        remaining base from   Sph I site
                                       /
|<---------pUC9 linker region----->|/
          10          20          30          40          50          60
   AAG.CTT.GGC.TGC.AGG.TCG.ACG.GAT.CCC.CGA.TGG.CGA.ACC.TGT.CGG.TAG.ACA.GCT.TGT.TTA.
   HindIII     PstI  SalI      BamHI   \
                                        \remaining bases from   Sma I site


          70          80          90          100         110         120
   TCG.AGT.ACG.ATG.ACC.GTC.GCC.CGG.TTG.GGC.TGG.AAC.CGT.TAC.GCC.AGG.TGA.GTT.GCC.AGA.


          130         140         150         160         170         180
   AGG.TGG.TAC.TGG.GGG.TCA.TCG.ATT.CCC.ACA.GTG.CTG.AGT.TGG.ATG.CGC.CGC.AGT.CAG.TCA.


          190         200         210         220         230         240
   GGA.TGG.CCA.TCA.ATA.CCG.CAT.CGT.TGT.ATC.TAC.CGT.TGC.AAC.AGT.TAG.CCA.TTA.GCC.CGA.
      BalI


          250         260         270         280         290         300
   AGT.GTG.GCT.TTG.GGC.ACT.GCG.AAC.AGC.AAG.GGA.TCA.CGG.AAG.AAC.AGC.AGT.GGG.CCA.AGT.


          310         320         330         340         350         360
   TAC.GGC.ATA.TGG.TGA.ATA.TCG.CTC.ATA.ACG.TCT.GGA.CCA.TGC.CGG.AAT.AGC.CTG.TCC.GGT.


          370         380         390         400         410         420
   AAA.ACA.ACG.CTG.TGG.CAT.CGC.GTC.AGC.GTT.GAT.GAC.ATG.CGC.CTG.AGC.CAG.GTT.CGT.CAA.


          430         440         450         460         470         480
   TCG.CTC.GTC.TTT.TCC.CTC.CCC.TGA.AAG.GTG.TTG.GTC.GAG.AAT.AGT.GAG.GTC.ATT.CAG.TGA.


          490         500         510         520         530         540
   TCT.GGC.TCT.CCT.GTT.GAT.CAT.CAG.GTG.CTA.TAC.ATA.ATT.CTT.ATG.GCA.CTG.CAA.CAT.AAC.
                BclI


          550         560         570         580         590         600
   CGC.CGA.TGC.CAG.TCC.TGC.AGA.CTG.GCA.GCT.AAT.GCG.TTT.TAC.GAG.AAA.AAA.TGA.TCG.GCT.
                          PstI
       [Pst site known to lie within or downstream of promotor, ie pASN42]


          610         620         630         640         650         660
   GGT.GCC.GGT.GTT.ATG.GCA.AAA.CAG.ACC.GAT.CAT.CCT.CTG.ACC.AAA.TAA.AGA.GAA.TCT.GTT.

   |-----------------------------------SIGNAL PEPTIDE------------------------
          670         680         690         700         710         720
   ATG.GAA.AGA.TGG.TTT.AAA.TCT.CTG.TTT.GTT.CTG.GTT.TTA.TTT.TTT.GTT.TTT.ACG.GCG.AGT.
   Met Glu Arg Trp Phe Lys Ser Leu Phe Val Leu Val Leu Phe Phe Val Phe Thr Ala Ser


   --|
```

# *FIG.4 (2)*

```
        730         740         750         760         770         780
GCG.GCA.GAT.AAA.CTG.CCC.AAT.ATC.GTT.ATC.CTG.GCG.ACC.GGC.GGT.ACA.ATT.GCC.GGC.TCA.
Ala Ala Asp Lys Leu Pro Asn Ile Val Ile Leu Ala Thr Gly Gly Thr Ile Ala Gly Ser
  ¦
  ¦N-terminus of Mature Protein


        790         800         810         820         830         840
GCG.GCA.ACG.GGT.ACC.CAA.ACC.ACA.GGT.TAC.AAG.GCT.GGC.GCG.CTT.GGC.GTG.GAT.ACG.CTA.
Ala Ala Thr Gly Thr Gln Thr Thr Gly Tyr Lys Ala Gly Ala Leu Gly Val Asp Thr Leu


        850         860         870         880         890         900
ATC.AAC.GCT.GTG.CCT.GAG.GTG.AAG.AAA.CTG.GCT.AAT.GTG.AAG.GGG.GAG.CAG.TTC.TCC.AAC.
Ile Asn Ala Val Pro Glu Val Lys Lys Leu Ala Asn Val Lys Gly Glu Gln Phe Ser Asn


        910         920         930         940         950         960
ATG.GCC.AGC.GAA.AAC.ATG.ACC.GGT.GAT.GTG.GTG.CTC.AAG.CTG.AGC.CAG.CGT.GTG.AAT.GAA.
Met Ala Ser Glu Asn Met Thr Gly Asp Val Val Leu Lys Leu Ser Gln Arg Val Asn Glu
 BalI


        970         980         990        1000        1010        1020
CTG.CTG.GCA.CGG.GAT.GAT.GTG.GAT.GGT.GTG.GTG.ATC.ACC.CAC.GGG.ACC.GAC.ACG.GTG.GAA.
Leu Leu Ala Arg Asp Asp Val Asp Gly Val Val Ile Thr His Gly Thr Asp Thr Val Glu
                                            BclI


       1030        1040        1050        1060        1070        1080
GAG.TCG.GCT.TAC.TTT.CTT.CAT.CTG.ACG.GTA.AAA.AGT.GAC.AAG.CCA.GTG.GTG.TTT.GTC.GCA.
Glu Ser Ala Tyr Phe Leu His Leu Thr Val Lys Ser Asp Lys Pro Val Val Phe Val Ala


       1090        1100        1110        1120        1130        1140
GCG.ATG.CGT.CCG.GCA.ACG.GCC.ATC.AGT.GCT.GAC.GGC.CCG.ATG.AAC.CTG.CTG.GAA.GCG.GTA.
Ala Met Arg Pro Ala Thr Ala Ile Ser Ala Asp Gly Pro Met Asn Leu Leu Glu Ala Val
                                ¦probable active site


       1150        1160        1170        1180        1190        1200
CGC.GTG.GCG.GGT.GAC.AAA.CAG.TCT.CGC.GGT.CGC.GGC.GTG.ATG.GTG.GTG.CTT.AAT.GAT.CGT.
Arg Val Ala Gly Asp Lys Gln Ser Arg Gly Arg Gly Val Met Val Val Leu Asn Asp Arg


       1210        1220        1230        1240        1250        1260
ATC.GGC.TCT.GCC.CGC.TAC.ATC.ACC.AAG.ACC.AAC.GCC.TCT.ACG.CTG.GAT.ACG.TTC.AAG.GCG.
Ile Gly Ser Ala Arg Tyr Ile Thr Lys Thr Asn Ala Ser Thr Leu Asp Thr Phe Lys Ala
```

# FIG.4 (3)

```
          1270        1280        1290        1300        1310        1320
AAT.GAA.GAA.GGC.TAC.CTG.GGC.GTC.ATT.ATT.GGT.AAC.CGC.ATT.TAC.TAC.CAA.AAC.CGT.ATC.
Asn Glu Glu Gly Tyr Leu Gly Val Ile Ile Gly Asn Arg Ile Tyr Tyr Gln Asn Arg Ile

          1330        1340        1350        1360        1370        1380
GAC.AAG.CTG.CAT.ACC.ACC.CGG.TCT.GTG.TTC.GAC.GTG.CGT.GGC.CTG.ACT.TCG.CTG.CCG.AAA.
Asp Lys Leu His Thr Thr Arg Ser Val Phe Asp Val Arg Gly Leu Thr Ser Leu Pro Lys

          1390        1400        1410        1420        1430        1440
GTC.GAC.ATT.CTT.TAT.GGC.TAT.CAG.GAT.GAC.CCG.GAA.TAT.CTG.TAT.GAC.GCG.GCT.ATC.CAG.
Val Asp Ile Leu Tyr Gly Tyr Gln Asp Asp Pro Glu Tyr Leu Tyr Asp Ala Ala Ile Gln
 SalI

          1450        1460        1470        1480        1490        1500
CAT.GGC.GTA.AAA.GGT.ATC.GTC.TAT.GCC.GGT.ATG.GGC.GCA.GGT.TCA.GTG.TCC.GTT.CGC.GGT.
His Gly Val Lys Gly Ile Val Tyr Ala Gly Met Gly Ala Gly Ser Val Ser Val Arg Gly

          1510        1520        1530        1540        1550        1560
ATT.GCC.GGT.ATG.CGC.AAG.GCG.ATG.GAG.AAA.GGC.GTT.GTT.GTG.ATC.CGT.TCT.ACC.CGC.ACA.
Ile Ala Gly Met Arg Lys Ala Met Glu Lys Gly Val Val Val Ile Arg Ser Thr Arg Thr

          1570        1580        1590        1600        1610        1620
GGC.AAT.GGT.ATT.GTG.CCG.CCG.GAT.GAA.GAG.CTG.CCA.GGT.CTG.GTT.TCT.GAC.TCT.CTT.AAC.
Gly Asn Gly Ile Val Pro Pro Asp Glu Glu Leu Pro Gly Leu Val Ser Asp Ser Leu Asn

          1630        1640        1650        1660        1670        1680
CCG.GCA.CAT.GCC.CGC.ATT.CTG.TTG.ATG.CTG.GCA.TTG.ACT.CGC.ACC.AGT.GAT.CCG.AAA.GTC.
Pro Ala His Ala Arg Ile Leu Leu Met Leu Ala Leu Thr Arg Thr Ser Asp Pro Lys Val

          1690        1700        1710        1720        1730        1740
ATT.CAA.GAG.TAT.TTC.CAT.ACT.TAT.TGA.TTG.TCT.TTA.TGT.TGA.TTG.CCT.TCG.CGT.TGA.TCG.
Ile Gln Glu Tyr Phe His Thr Tyr ***
                            |translation termination codon

          1750        1760        1770        1780        1790        1800
CTT.TAG.CGT.GTG.AGT.GCG.ATT.TGA.ACT.ATG.CAG.GCC.GCC.TTT.GGG.CGG.CCT.TAT.TGT.TGG.
                            Transcription terminator

          1810        1820        1830        1840        1850        1860
TAA.CAG.AAC.CAT.CAA.TAG.CAG.TCG.ATG.TTT.CAT.GGG.AAG.ATT.AGC.CAT.GTC.TGG.TGT.AAA.

          1870        1880        1890        1900        1910        1920
TCG.TGG.CGT.GTT.GTG.CTG.GAT.TTG.TGC.GGA.AAG.CTG.GCG.TGA.AGT.GCC.TTT.CCC.CGT.ACC.

          1930        1940        1950        1960        1970        1980
AGA.GCC.GTA.GCG.AGG.CTT.TCT.TAA.TGA.GGA.TTA.TTG.CCT.GTA.TGA.TTC.TGC.TCT.TGG.TAC.
```

# FIG.4(4)

```
      1990          2000          2010          2020          2030          2040
GAC.AGG.CGC.TGG.CTG.ACA.GCA.ACG.TCG.TTG.CGA.AGA.GCT.TGC.CTG.AGA.AAA.AGA.AGG.ACA.

      2050          2060          2070          2080          2090          2100
GGT.CGT.GAC.ATG.ACA.GAA.CCC.ATT.TTT.ATG.GTC.GGC.GCC.AGA.GGG.TGC.GGA.AAA.ACC.ACC.

      2110          2120          2130          2140          2150          2160
GTC.GGC.CGT.GAG.CTG.GCG.CGG.GCG.CTT.GGG.TAT.GAG.TTT.GTC.GAT.ACG.GAT.ATT.TTT.ATG.

      2170          2180          2190          2200          2210          2220
CAG.CAC.ACC.AGC.GGC.ATG.ACA.GTG.GCG.GAC.GTG.GTG.GCA.GCG.GAA.GGC.TGG.CCG.GGG.TTT.

      2230          2240          2250          2260          2270          2280
CGC.CGC.CGT.GAA.AGC.GAG.GCC.TTG.CAG.GCT.GTC.GCG.ACA.CCG.AAT.CGT.GTG.GTG.GCG.ACC.

      2290          2300          2310          2320          2330          2340
GGT.GGC.GGC.ATG.GTG.TTG.TTG.GAG.CAA.AAC.CGC.CAG.TTT.ATG.CGT.GCG.CAC.GGA.ACG.GTC.

      2350          2360          2370          2380          2390          2400
GTC.TAT.TTG.TTC.GCG.CCA.GCC.GAG.GAG.CTG.GCG.TTA.CGC.TTG.CAG.GCA.AGC.CCA.CAG.GCA.

      2410          2420          2430          2440          2450          2460
GAC.CAG.CGA.CCG.ACA.CTG.ACC.GGG.CGG.CCT.ATT.GCT.GAG.GAA.ATG.GAG.GCG.GTG.CTC.CGT.

      2470          2480          2490          2500          2510          2520
GAA.CGC.GAA.GCG.TTG.TAT.CAG.GAT.GTA.GCG.CAC.TAT.GTG.GTC.GAT.GCG.ACC.CAA.CCG.CCC.

      2530          2540          2550          2560          2570          2580
GCC.GCG.ATT.GTC.TGC.GAA.TTG.ATG.CAG.ACG.ATG.CGT.CTG.CCT.GCG.GCC.TGA.CGG.ACA.ACT.

      2590          2600          2610          2620          2630          2640
GAT.TTT.TTA.ACA.GAA.GGG.TTG.GCA.TTA.TGC.TGA.ATC.TAA.ATG.AGT.ATT.TTG.AAG.GAA.AAG.

      2650          2660          2670          2680          2690          2700
TAA AAT CCA TCG GGT TTG ACG GTA GCC GCA TCG GTC GTG  CCA GCG TCG GGG TGA TGG AAG

      2710          2720          2700          2740          2750          2760
CAG.GCG.AAT.ACA.CGT.TTG.GTA.CCG.GTC.AGG.CGG.AAG.AAA.TGA.CCG.TCG.TTA.GCG.GTG.CGT.

      2770          2780          2760          2800          2810          2820
TGA.ACG.TGT.TGC.TGC.CGG.AGT.ACA.GGA.ATG.GCA.GTT.GTT.CGA.AGC.CGG.TGC.TGT.GTT.CAA.

      2830          2840
CGT.GCC.GGA.AAA.AGC.GA        -------> Eco RI site few bases down stream
```